(19) Europäisches Patentamt
European Patent Office
Office européen des brevets

(11) Veröffentlichungsnummer: **0 429 902 A1**

# (12) EUROPÄISCHE PATENTANMELDUNG

(21) Anmeldenummer: **90121258.9**

(51) Int. Cl.[5]: **C07D 319/04**, A61K 7/44

(22) Anmeldetag: **07.11.90**

(30) Priorität: **18.11.89 DE 3938468**

(43) Veröffentlichungstag der Anmeldung: **05.06.91 Patentblatt 91/23**

(84) Benannte Vertragsstaaten: **BE CH DE ES FR GB IT LI NL Patentblatt**

(71) Anmelder: **BASF Aktiengesellschaft**
**Carl-Bosch-Strasse 38**
**W-6700 Ludwigshafen(DE)**

(72) Erfinder: **Guembel, Helmut, Dr.**
**Altrheinstrasse 56**
**W-6700 Ludwigshafen(DE)**
Erfinder: **Paust, Joachim, Dr.**
**Ringstrasse 3**
**W-6708 Neuhofen(DE)**
Erfinder: **Sperling-Vietmeier, Karin, Dr.**
**Im Kirchenstueck 12**
**W-6730 Neustadt(DE)**
Erfinder: **Becker, Rainer, Dr.**
**Im Haseneck 22**
**W-6702 Bad Duerkheim(DE)**

## (54) 1,1-Dialkoxy-2-oxo-6-aryl-3,5-alkadiene.

(57) 1,1-Dialkoxy-2-oxo-6-aryl-3,5-alkadiene der allgemeinen Formel I

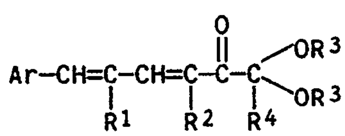

I

in der die Substituenten folgende Bedeutung haben:
Ar ein Phenyl, Biphenyl- oder Naphthylrest, der durch ein bis drei $C_1$-$C_4$-Alkylgruppen, $C_1$-$C_4$-Alkoxygruppen, Hydroxylgruppen, Phenoxygruppen, gegebenenfalls durch $C_1$-$C_4$-Alkylgruppen mono- oder disubstituierte Aminogruppen, Halogenatome oder eine Methylendioxygruppe substituiert sein kann, wobei die Substituenten gleich oder verschieden sein können,
$R^1$ Wasserstoff oder eine $C_1$-$C_8$-Alkylgruppe,
$R^2$ Wasserstoff, eine $C_1$-$C_4$-Alkylgruppe, eine $C_1$-$C_{10}$-Alkoxycarbonylgruppe oder eine $C_1$-$C_{10}$-Acylgruppe,
$R^3$ $C_1$-$C_8$-Alkylgruppen, die unter Ausbildung eines fünf- oder sechsgliedrigen Ringes miteinander verbunden sein können, und
$R^4$ Wasserstoff oder eine $C_1$-$C_4$-Alkylgruppe.

Die Verbindungen I dienen als Lichtschutzmittel in kosmetischen Zubereitungen.

EP 0 429 902 A1

## 1,1-DIALKOXY-2-OXO-6-ARYL-3,5-ALKADIENE

Die vorliegende Erfindung betrifft 1,1-Dialkoxy-2-oxo-6-aryl-3,5-alkadiene der allgemeinen Formel I

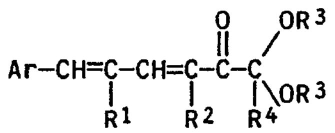

I

in der die Substituenten folgende Bedeutung haben:
Ar ein Phenyl-, Biphenyl- oder Naphthylrest, der durch ein bis drei $C_1$-$C_4$-Alkylgruppen, $C_1$-$C_4$-Alkoxygruppen, Hydroxylgruppen, Phenoxygruppen, Aminogruppen, welche durch $C_1$-$C_4$-Alkylgruppen mono- oder disubstituiert sein können, Halogenatome oder eine Methylendioxygruppe substituiert sein kann, wobei die Substituenten gleich oder verschieden sein können,
$R^1$ Wasserstoff oder eine $C_1$-$C_8$-Alkylgruppe,
$R^2$ Wasserstoff, eine $C_1$-$C_4$-Alkylgruppe, eine $C_1$-$C_{10}$-Alkoxycarbonylgruppe oder eine $C_1$-$C_{10}$-Acylgruppe,
$R^3$ $C_1$-$C_8$-Alkylgruppen, die unter Ausbildung eines fünf- oder sechsgliedrigen Ringes miteinander verbunden sein können, und
$R^4$ Wasserstoff oder eine $C_1$-$C_4$-Alkylgruppe.

Außerdem betrifft die Erfindung ein Verfahren zur Herstellung der Verbindungen I sowie ihre Verwendung als Lichtschutzmittel in kosmetischen Zubereitungen.

Aus der DE-A 27 28 242 sind Brenztraubensäure- und Lävulinsäure-Kondensationsprodukte der allgemeinen Formel IV bekannt

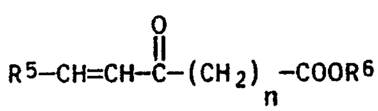

IV

in der $R^5$ Arylreste und 2-Arylvinylreste bedeutet, $R^6$ Wasserstoff, $C_1$-$C_4$-Alkyl oder ein salzbildendes Kation bezeichnet und n 0 oder 2 ist. Die Verbindungen IV dienen als kosmetische Lichtschutzmittel für den UV-A-Bereich.

In der DE-C 32 06 586 werden 2-Acyl-5-phenyl-2,4-pentadiensäureester der allgemeinen Formel V

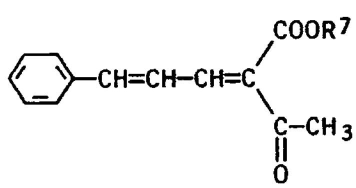

V

in der $R^7$ für einen $C_1$-$C_8$-Alkylrest oder einen Alkoxyalkylrest mit insgesamt 4 C-Atomen steht, beschrieben. Die Ester V werden als Lichtschutzmittel für kosmetische und technische Anwendungen empfohlen.

Die US-A 4,515,774 betrifft Cinnamylidenmalonsäuredialkylester der allgemeinen Formel VI

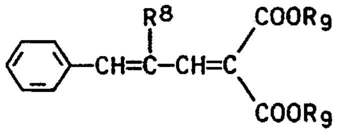

VI

in der $R^8$ Wasserstoff oder eine $C_1$-$C_8$-Alkylgruppe bezeichnet und $R^9$ für Methyl oder Ethyl steht. Die Verbindungen VI dienen als Sonnenschutzmittel für die menschliche Haut.

Es besteht ein wachsender Bedarf an Lichtschutzmitteln für kosmetische Zubereitungen, die als UV-A-Filter dienen können und deren Absorptionsmaxima deshalb im Bereich von ca. 330 bis 380 nm liegen

sollten, wie es bei den Verbindungen IV bis VI der Fall ist. Um mit einer möglichst geringen Einsatzmenge die gewünschte Wirkung zu erzielen, sollten derartige Lichtschutzmittel zusätzlich eine hohe spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen sowie geringen Eigengeruch und geringe Eigenfärbung. Die Verbindungen IV bis VI genügen diesen Anforderungen an UV-A-Filtersubstanzen für kosmetische Anwendungen jedoch nur bedingt.

Der vorliegenden Erfindung lag daher die Aufgabe zugrunde, Lichtschutzmittel für kosmetische Zubereitungen bereitzustellen, die allen genannten Anforderungen in höherem Maße gerecht werden als die bekannten Mittel dieser Art.

Demgemäß wurden die 1,1-Dialkoxy2-oxo-6-aryl-3,5-alkadiene I gefunden.

Für den Arylrest Ar kommt weiterhin insbesondere ein Phenylrest, der durch ein bis drei $C_1$-$C_4$-Alkylgruppen oder durch ein oder zwei $C_1$-$C_4$-Alkoxygruppen, Hydroxylgruppen oder Di($C_1$-$C_4$-alkyl)-aminogruppen substituiert sein kann, oder ein unsubstituierter Biphenyl- oder Naphthylrest in Betracht.

Als Beispiele für Ar sind zu nennen:

Phenyl,
o-, m- oder p-Tolyl,
o-, m- oder p-Ethylphenyl,
m- oder p-Cumyl,
m- oder p-tert.-Butylphenyl,
2,3-, 2,4-, 2,5-, 2,6-, 3,4- oder 3,5-Dimethylphenyl,
Mesityl,
o-, m- oder p-Methoxyphenyl,
o-, m- oder p-Ethoxyphenyl,
m- oder p-tert.-Butoxyphenyl,
2,3-, 2,4-, 2,5-, 2,6-, 3,4- oder 3,5-Dimethoxyphenyl,
o-, m- oder p-Hydroxyphenyl,
2,3-, 2,4-, 2,5-, 2,6-, 3,4- oder 3,5-Dihydroxyphenyl,
3-Hydroxy-4-methoxyphenyl,
m- oder p-Phenoxyphenyl,
o-, m- oder p-Aminophenyl,
o-, m- oder p-(N-Methylamino)phenyl,
o-, m- oder p-(N,N-Dimethylamino)phenyl,
o-, m- oder p-Chlorphenyl,
2,4-Dichlorphenyl,
o-, m- oder p-Bromphenyl,
2,3- oder 3,4-Methylendioxyphenyl,
2-, 3- oder 4-Biphenyl und
$\alpha$- oder $\beta$-Naphthyl.

Der Rest $R^1$ steht bevorzugt für Wasserstoff oder eine $C_1$-$C_5$-Alkylgruppe, beispielsweise Methyl, Ethyl, n-Propyl, n-Butyl oder n-Pentyl. Besonders bevorzugt wird Wasserstoff.

Der Rest $R^2$ bedeutet bevorzugt Wasserstoff, eine $C_1$-$C_4$-Alkylgruppe wie Methyl, Ethyl, n-Propyl oder n-Butyl, eine $C_1$-$C_4$-Alkoxycarbonylgruppe, z.B. Methoxycarbonyl oder Ethoxycarbonyl, oder eine $C_1$-$C_4$-Acylgruppe wie Acetyl, Propionyl oder Butyryl. Besonders bevorzugt wird Wasserstoff oder eine $C_1$-$C_4$-Alkylgruppe.

Die Acetalreste $R^3$ können verschieden oder vorzugsweise gleich sein und bezeichnen $C_1$-$C_8$-Alkylgruppen, vorzugsweise $C_1$-$C_4$-Alkylgruppen, wie Methyl, Ethyl, n-Propyl, n-Butyl oder 2-Ethylhexyl; die beiden Reste $R^3$ können unter Ausbildung eines fünf- oder sechsgliedrigen Ringes, zum Beispiel eines 1,3-Dioxolan-, 1,3-Dioxan-, 5,5-Dimethyl-1,3-dioxan- oder 5-n-Butyl-5-ethyl-1,3-dioxan-Ringes, miteinander verbunden sein.

Der Rest $R^4$ steht bevorzugt für Wasserstoff oder eine Methylgruppe, daneben kann er aber auch eine $C_2$-$C_4$-Alkylgruppe bezeichnen, beispielsweise eine Ethyl-, n-Propyl- oder n-Butylgruppe.

Die erfindungsgemäßen Verbindungen I können als cis-trans-Isomerengemische bezüglich der beiden olefinischen Doppelbindungen oder als isomerenreine Verbindungen vorliegen. Auf ihre kosmetische Verwendungsmöglichkeit hat die cis-trans-Isomerie der Verbindungen I keinerlei Einfluß.

Die erfindungsgemäßen Verbindungen I werden zweckmäßigerweise durch Aldol-Kondensation eines Aldehyds der allgemeinen Formel II

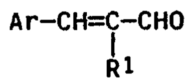

II

mit einem Methylglyoxalmonoacetal-Derivat der allgemeinen Formel III

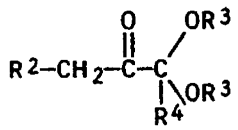

III

in an sich bekannter Weise unter basischen Reaktionsbedingungen hergestellt.

Die Umsetzung wird normalerweise in einem organischen Lösungsmittel durchgeführt, beispielsweise in einem Alkohol wie Methanol, Ethanol, Isopropanol oder n-Butanol, einem Ether wie Diethylether, Methyl-tert.-butylether, Diisopropylether, Tetrahydrofuran oder Dioxan oder einem Kohlenwasserstoff wie Toluol oder Xylol. Als Base können beispielsweise Natriumhydroxid, Kaliumhydroxid, Natriummethylat, Natriumethylat, Natrium-tert.-butylat, Kalium-tert.-butylat, Natriumamid, Natriumdimethylamid, Natriumdiisopropylamid, Lithiumdiisopropylamid oder Natriumhydrid verwendet werden. In der Regel arbeitet man bei Normaldruck und bei Temperaturen von 0 bis 150 ° C, insbesondere von 0 bis 80 ° C.

Die erfindungsgemäßen Verbindungen I dienen als Lichtschutzmittel in kosmetischen Zübereitungen zur vorsorglichen Sonnenschutzpflege. Solche Sonnenschutzpräparate können in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Gegenstand der vorliegenden Erfindung sind weiterhin kosmetische Zubereitungen, die 0,1 bis 10 Gew.-%, vorzugsweise 1 bis 7 Gew.-%, bezogen auf die gesamte kosmetische Zubereitung, einer oder mehrerer der erfindungsgemäßen Verbindungen I als Lichtschutzmittel enthalten, wobei die Verbindungen I in üblichen Trägerstoffen oder Verdünnungsmitteln eingesetzt werden, beispielsweise als Lösung in einem Öl.

Übliche Ölkomponenten in der Kosmetik sind beispielsweise Paraffinöl, Glycerylstearat, Isopropylmyristat, Diisopropyladipat, 2-Ethylhexansäure-cetylstearylester, hydriertes Polyisobuten, Vaseline, Caprylsäure/Caprinsäure-Triglyceride, mikrokristallines Wachs, Lanolin und Stearinsäure.

Zusammen mit den Verbindungen I können weitere übliche Lichtschutzmittel wie
2-Hydroxy-4-methoxybenzophenon,
2-Hydroxy-4-methoxybenzophenon-5-sulfonsäure,
mit 25 mol Ethylenoxid umgesetzter p-Aminobenzoesäureethylester,
p-Methoxyzimtsäure-2-ethylhexylester,
p-(N,N-Dimethylamino)benzoesäure-2-ethylhexylester,
2-Phenylbenzimidazol-5-sulfonsäure,
3-(4methylbenzyliden)campher oder
2,4,6-Trianilino-p-(carbo-2′-ethylhexyl-1′-oxy)-1,3,5-triazin
in den hierfür üblichen Mengen mitverwendet werden, wobei sogar eine Potenzierung der Lichtschutzwirkung durch einen synergistischen Effekt auftreten kann.

Übliche kosmetische Hilfsstoffe, die als Zusätze in Betracht kommen können, sind z.B. Emulgatoren wie Fettalkoholethoxylate, Sorbitanfettsäureester oder Lanolinderivate, Verdickungsmittel wie Carboxymethylcellulose oder vernetzte Polyacrylsäure, Konservierungsmittel und Parfüms.

Die erfindungsgemäßen 1,1-Dialkoxy-2-oxo-6-aryl-3,5-alkadiene I zeichnen sich durch ein besonders hohes Absorptionsvermögen im Bereich der UV-A-Strahlung aus. Sie können auch vorteilhaft in Kombination mit UV-B-Filtersubstanzen verwendet werden. Weiterhin sind sie gut in kosmetischen Ölen löslich und lassen sich leicht in kosmetische Formulierungen einarbeiten. Die mit den erfindungsgemäßen Verbindungen hergestellten Emulsionen zeichnen sich besonders durch ihre hohe Stabilität und ihr angenehmes Hautgefühl aus.

Die Verbindungen I sind nahezu farblos und geruchlos. Derzeitig verfügbare, handelsübliche UV-A-Filtersubstanzen sind gelb und neigen dazu, auch auf Kleidungsstücke abzufärben. Dies kann durch Verwendung der erfindungsmäßigen Verbindungen vermieden werden.

Herstellungsbeispiele
Beispiel 1

5,5-Dimethyl-2-(2-methyl-5-phenyl-2,4-pentadienoyl)-1,3-dioxan

Zu einer Lösung von 52,9 g Zimtaldehyd und 68,9 g 5,5-Dimethyl-2-propionyl-1,3-dioxan in 200 ml Methanol wurden innerhalb von 15 min bei 0-5° C 7,2 g einer 30 gew.-%igen Lösung von Natriummethylat in Methanol zugetropft und weitere 2 h bei 0-5° C gerührt. Anschließend wurde die Reaktionslösung mit einigen Kristallen des Produktes angeimpft und über Nacht bei Raumtemperatur gerührt. Nach Absaugen und Waschen des Niederschlages mit kaltem Methanol wurden 29,8 g des Produktes in Form von farblosen Kristallen von Schmelzpunkt 122-125° C erhalten.

Spektroskopische Daten: IR $\nu$ (C=O) = 1661 $cm^{-1}$

$$UV\ \lambda_{max} = 332\ nm,\ E_{1\ cm}^{1\ \%} = 1380$$

Beispiel 2

2-Methyl-2-(5-phenyl-2,4-pentadienoyl)-1,3-dioxolan

Zu einer Lösung von 26,4 g Zimtaldehyd und 26,0 g 2-Acetyl-2-methyl-1,3-dioxolan in 100 ml Methanol wurden innerhalb von 10 min bei 20 bis 25° C 4,0 g einer 15 gew.-%igen Kalilauge zugetropft und weitere 4 h bei 20 bis 25° C gerührt. Anschließend wurde das Lösungsmittel abdestilliert, der Rückstand in 200 ml tert.-Butylmethylether aufgenommen und die Lösung mit Wasser gewaschen und über Natriumsulfat getrocknet. Nach Abdestillieren des Lösungsmittels wurde das Rohprodukt bei 150 bis 155° C bei einem Druck von 0,04 mbar destilliert.

Man erhielt 12,0 g des Produktes in Form eines blaßgelben Öls.

Spektroskopische Daten: IR $\nu$ (C=O) = 1691 $cm^{-1}$

$$UV\ \lambda_{max} = 331\ nm,\ E_{1\ cm}^{1\ \%} = 1290$$

Anwendungsbeispiele

Beispiel 3: Wasser-in-Öl-Creme

Eine Wasser-in-Öl(W/O)-Lichtschutzcreme weist folgende Zusammensetzung auf:

12,0 g Diisopropyladipat
10,0 g Isopropylmyristat
8,0 g ungesättigter Glycerolsorbitanfettsäureester
5,0 g Vaseline
5,0 g 1,2-Propylenglykol
2,0 g der Verbindung aus Beispiel 1
2,0 g mit 7 mol Ethylenoxid umgesetztes hydriertes Rizinusöl
2,0 g mit 45 mol Ethylenoxid umgesetztes Dodecanol
2,0 g Mikrokristallines Wachs
0,7 g Magnesiumsulfat-heptahydrat
0,5 g Magnesiumstearat
0,5 g Aluminiumstearat
0,3 g Konservierungsmittel
50,0 g Wasser

Beispiel 4: Öl-in-Wasser-Creme

Eine Öl-in-Wasser(O/W)-Lichtschutzcreme weist folgende Zusammensetzung auf:

12,0 g Glycerylstearat
10,0 g Caprylsäure/Caprinsäure-Triglycerid
10,0 g Diisopropyladipat
3,0 g Glycerin
2,0 g der Verbindung aus Beispiel 1
1,0 g Mischung aus einem höheren gesättigten Fettalkohol, mit 6 mol Ethylenoxid umgesetzt, und Stearylalkohol
1,0 g höherer gesättigter Fettalkohol, mit 25 mol Ethylenoxid umgesetzt
0,5 g Konservierungsmittel
60,5 g Wasser

## Ansprüche

1. 1,1-Dialkoxy-2-oxo-6-aryl-3,5-alkadiene der allgemeinen Formel I

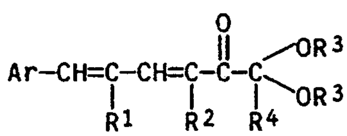

I

in der die Substituenten folgende Bedeutung haben:

Ar ein Phenyl-, Biphenyl- oder Naphthylrest, der durch ein bis drei $C_1$-$C_4$-Alkylgruppen, $C_1$-$C_4$-Alkoxygruppen, Hydroxylgruppen, Phenoxygruppen, Aminogruppen, welche durch $C_1$-$C_4$-Alkylgruppen mono- oder disubstituiert sein können, Halogenatome oder eine Methylendioxygruppe substituiert sein kann, wobei die Substituenten gleich oder verschieden sein können,

$R^1$ Wasserstoff oder eine $C_1$-$C_8$-Alkylgruppe,

$R^2$ Wasserstoff, eine $C_1$-$C_4$-Alkylgruppe, eine $C_1$-$C_{10}$-Alkoxycarbonylgruppe oder eine $C_1$-$C_{10}$-Acylgruppe,

$R^3$ $C_1$-$C_8$-Alkylgruppen, die unter Ausbildung eines fünf- oder sechsgliedrigen Ringes miteinander verbunden sein können, und

$R^4$ Wasserstoff oder eine $C_1$-$C_4$-Alkylgruppe.

2. Verbindungen I nach Anspruch 1, bei denen die Substituenten folgende Bedeutung haben:

Ar ein Phenylrest, der durch ein bis drei $C_1$-$C_4$-Alkylgruppen oder durch ein oder zwei $C_1$-$C_4$-Alkoxygruppen, Hydroxylgruppen oder Di($C_1$-$C_4$-alkyl)aminogruppen substituiert sein kann, oder ein unsubstituierter Biphenyl- oder Naphthylrest,

$R^1$ Wasserstoff oder eine $C_1$-$C_5$-Alkylgruppe,

$R^2$ Wasserstoff, eine $C_1$-$C_4$-Alkylgruppe, eine $C_1$-$C_4$-Alkoxycarbonylgruppe oder eine $C_1$-$C_4$-Acylgruppe,

$R^3$ $C_1$-$C_4$-Alkylgruppen, die unter Ausbildung eines fünf- oder sechsgliedrigen Ringes miteinander verbunden sein können, und

$R^4$ Wasserstoff oder eine $C_1$-$C_4$-Alkylgruppe.

3. Verfahren zur Herstellung der Verbindungen I gemäß Anspruch 1 oder 2, dadurch gekennzeichnet, daß man einen Aldehyd der allgemeinen Formel II

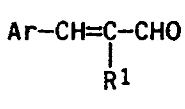

II

mit einem Methylglyoxalmonoacetal-Derivat der allgemeinen Formel III

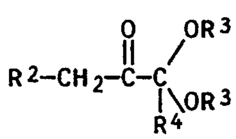

III

in an sich bekannter Weise unter basischen Reaktionsbedingungen umsetzt.

4. Verwendung der Verbindungen I gemäß Anspruch 1 oder 2 als Lichtschutzmittel in kosmetischen Zubereitungen.

5. Kosmetische Zubereitungen, enthaltend 0,1 bis 10 Gew.-% der Verbindungen I gemäß Anspruch 1 oder 2 als Lichtschutzmittel.

Patentansprüche für folgenden Vertragsstaat: ES

1. Verfahren zur Herstellung von 1,1-Dialkoxy-2-oxo-6-aryl-3,5-alkadienen der allgemeinen Formel I

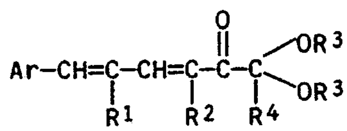

I

in der die Substituenten folgende Bedeutung haben:

Ar ein Phenyl-, Biphenyl- oder Naphthylrest, der durch ein bis drei $C_1$-$C_4$-Alkylgruppen, $C_1$-$C_4$-Alkoxygruppen, Hydroxylgruppen, Phenoxygruppen, Aminogruppen, welche durch $C_1$-$C_4$-Alkylgruppen mono- oder disubstituiert sein können, Halogenatome oder eine Methylendioxygruppe substituiert sein kann, wobei die Substituenten gleich oder verschieden sein können,

$R^1$ Wasserstoff oder eine $C_1$-$C_8$-Alkylgruppe,

$R^2$ Wasserstoff, eine $C_1$-$C_4$-Alkylgruppe, eine $C_1$-$C_{10}$-Alkoxycarbonylgruppe oder eine $C_1$-$C_{10}$-Acylgruppe,

$R^3$ $C_1$-$C_8$-Alkylgruppen, die unter Ausbildung eines fünf- oder sechsgliedrigen Ringes miteinander verbunden sein können, und

$R^4$ Wasserstoff oder eine $C_1$-$C_4$-Alkylgruppe,

dadurch gekennzeichnet, daß man einen Aldehyd der allgemeinen Formel II

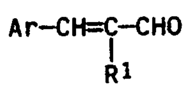

II

mit einem Methylglyoxalmonoacetal-Derivat der allgemeinen Formel III

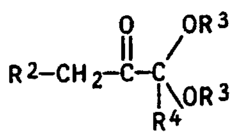

III

in an sich bekannter Weise unter basischen Reaktionsbedingungen umsetzt.

2. Verfahren nach Anspruch 1, dadurch gekennzeichnet, daß man es auf die Herstellung von Verbindungen I anwendet, bei denen die Substituenten folgende Bedeutung haben:

Ar ein Phenylrest, der durch ein bis drei $C_1$-$C_4$-Alkylgruppen oder durch ein oder zwei $C_1$-$C_4$-Alkoxygruppen, Hydroxylgruppen oder Di($C_1$-$C_4$-alkyl)aminogruppen substituiert sein kann, oder ein unsubstituierter Biphenyl- oder Naphthylrest,

$R^1$ Wasserstoff oder eine $C_1$-$C_5$-Alkylgruppe,

$R^2$ Wasserstoff, eine $C_1$-$C_4$-Alkylgruppe, eine $C_1$-$C_4$-Alkoxycarbonylgruppe oder eine $C_1$-$C_4$-Acylgruppe,

$R^3$ $C_1$-$C_4$-Alkylgruppen, die unter Ausbildung eines fünf- oder sechsgliedrigen Ringes miteinander verbunden sein können, und

$R^4$ Wasserstoff oder eine $C_1$-$C_4$-Alkylgruppe.

3. Verfahren zur Herstellung von kosmetischen Zubereitungen, dadurch gekennzeichnet, daß man 0,1 bis 10 Gew.-% oder Verbindungen I gemäß Anspruch 1 oder 2, bezogen auf die Gesamtmenge der Zubereitung, als Lichtschutzmittel in die kosmetischen Zubereitungen einarbeitet.

Europäisches Patentamt

# EUROPÄISCHER RECHERCHENBERICHT

Nummer der Anmeldung

EP 90121258.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | DE - C1 - 3 206 586 (DRAGOCO GERBERDING & CO. GMBH) * Ansprüche 1,2 * -- | 1-5 | C 07 D 319/04<br>A 61 K 7/44 |
| D,A | DE - A1 - 2 728 242 (HENKEL KGAA) * Ansprüche 1-3 * -- | 1-5 | |
| A | CHEMICAL ABSTRACTS, Band 109, Nr. 19, 7. November 1988, Columbus, Ohio, USA TERUMO CORP "5-Phenyl-2,4--pentadien-1-one derivatives and their use as platelet aggregation inhibitors and lipoxygenase inhibitors" Seite 680, Spalte 2, Zusammenfassung-Nr. 170 035b & Jpn. Kokai Tokkyo Koho JP 62,281,839 (87 281 839) -- | 1 | |
| | | | RECHERCHIERTE SACHGEBIETE (Int Cl 5) |
| A | CHEMICAL ABSTRACTS, Band 106, Nr. 25, 22. Juni 1987, Columbus, Ohio, USA TERUMO CORP "1-(Imidazol-1--yloxoalkenyl)-3,4-dihydroxy benzene derivatives as thromboxene A2 synthase inhibitors" Seite 665, Spalte 2, Zusammenfassung-Nr. 213 952f & Jpn. Kokai Tokkyo Koho JP 62,42,972 (87 42 972) ---- | 1 | C 07 D 319/00 |

Der vorliegende Recherchenbericht wurde für alle Patentansprüche erstellt.

| Recherchenort | Abschlußdatum der Recherche | Prüfer |
|---|---|---|
| WIEN | 06-12-1990 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument